# FASCICULE DE BREVET EUROPEEN

(11) **EP 1 536 808 B1**
(45) Date de publication et mention de la délivrance du brevet: **08.07.2009**
(21) Numéro de dépôt: 03758268.1
(22) Date de dépôt: 25.08.2003
(51) Int. Cl.: A61K 36/75, A61K 38/16, A61P 35/00

(54) **L'HETEROCARPINE, UNE PROTEINE D ORIGINE VEGETALE AUX PROPRIETES ANTICANCEREUSES**
HETEROCARPINE, EIN PFLANZLICHES PROTEIN MIT ANTIKREBS EIGENSCHAFTEN
HETEROCARPIN, A PLANT-DERIVED PROTEIN WITH ANTI-CANCER PROPERTIES

(30) Priorité: 26.08.2002 FR 0210560
(43) Date de publication de la demande: 08.06.2005
(73) Titulaire: IPSEN PHARMA, 92100 Boulogne-Billancourt (FR)
(72) Inventeur: FERRANDIS, Eric, F-78470 Saint Rémy les Chevreuse (FR); TENG, Beng, Poon, F-91190 Gif-sur-Yvette (FR); SOHIER, Christine, F-37390 Saint Roch (FR); THURIEAU, Christophe, F-75116 Paris (FR)
(74) Mandataire: Bourgouin, André
(86) Numéro de dépôt international: PCT/FR2003/002570
(87) Numéro de publication internationale: WO 2004/017987

(56) Documents cités:
- WO-A-95/16707
- WO-A-02/068461
- JAFFE C A ET AL: "Suppression of growth hormone (GH) hypersecretion due to ectopic GH-releasing hormone (GHRH) by a selective GHRH antagonist" JOURNAL OF CLINICAL ENDOCRINOLOGY AND METABOLISM, NEW YORK, NY, US, vol. 82, no. 2, 1997, pages 634-637, XP002182305 ISSN: 0021-972X

## Description

La présente invention concerne l'utilisation une protéine d'origine végétale aux propriétés anticancéreuses qui fixe le GHRH humain (*human Growth Hormone releasing hormone* ou hormone libératrice d'hormone de croissance humaine).

L'hormone de croissance (« GH ») est une protéine de 191 acides aminés qui stimule la production de nombreux facteurs de croissance, comme l'*Insulin-Like Growth Factor I* (IGF-1) et déclenche la croissance d'un grand nombre de tissus (squelette, tissus connectifs, muscles et viscères). GH possède également des activités physiologiques en augmentant la synthèse des acides nucléiques, des protéines et de la lipolyse tout en diminuant les sécrétions urinaires (Frohman L.A. & Kineman, R.D., Handbook of Physiology, Hormonal Control of Growth, édité par Kostyo, J.L. & Goodman, H.M. (Oxford Univ. Press, New York, 1999), p. 189-221).

La synthèse de GH est régulée par des facteurs à action positive ou négative sécrétés par l'hypothalamus. Le facteur majoritaire contrôlant la production de GH est le « Growth Hormone Releasing Hormone » (GHRH), peptide de 44 acides aminés chez l'homme.

GH et GHRH sont impliqués dans de nombreuses maladies. Parmi celles-ci, il y a lieu de citer notamment le cancer (en particulier ceux de la prostate ou du poumon), l'acromégalie, les rétinopathies et les néphropathies diabétiques ; pour ces pathologies, un traitement par des antagonistes de GHRH est indiqué. Du fait du nombre de maladies potentiellement concernées, l'industrie continue à chercher des antagonistes de GHRH.

La demanderesse vient donc justement d'isoler une nouvelle protéine d'origine végétale, laquelle a pour propriété de fixer le GHRH humain.

L'invention concerne une protéine isolée susceptible d'être obtenue par extraction de la plante *Pilocarpus heterophyllus*, laquelle est caractérisée en ce qu'elle possède une masse moléculaire d'environ 90,9 kDa et comporte les fragments de séquences peptidiques SEQ. ID. NO. 1, SEQ. ID. NO. 2 et SEQ. ID. NO. 3, ladite protéine étant susceptible de se présenter sous une forme glycosylée ou non glycosylée. Pour simplifier l'exposé qui suit, cette protéine sera désignée ci-après par « hétérocarpine ».

Lesdites séquences SEQ. ID. NO. 1, SEQ. ID. NO. 2 et SEQ. ID. NO. 3 sont les suivantes :

| | |
|---|---|
| SEQ. ID. NO. 1 : | KLIGARYFDK |
| SEQ. ID. NO. 2 : | YGEDIRVGVIDSGV |
| SEQ. ID. NO. 3 : | PESESY |

La nomenclature utilisée ci-dessus (comme dans le reste de la présente demande) pour définir les peptides est celle spécifiée par la « IUPAC-IUB Commissioner on Biochemical Nomenclature » dans laquelle, en accord avec la représentation conventionnelle, l'acide aminé au niveau N-terminal (groupe amino) apparaît à gauche et l'acide aminé au niveau C-terminal (groupe carboxyle) apparaît à droite. Le terme « acide aminé naturel » indique l'un des L-acides aminés naturels trouvé dans les protéines naturelles : Gly, Ala, Val, Leu, Ile, Ser, Thr, Lys, Arg, Asp, Asn, Glu, Gln, Cys, Met, Phe, Tyr, Pro, Trp et His.

Une protéine est dite « isolée » si elle est prise hors de son environnement original. En particulier, une protéine naturelle est isolée si elle est séparée du matériel biologique avec lequel elle coexiste dans le système naturel.

L'invention concerne de préférence l'hétérocarpine sous sa forme non glycosylée.

Selon une variante préférée de l'invention, l'hétérocarpine est obtenue à partir d'un extrait des cellules de la plante *Pilocarpus Heterophyllus* cultivées *in vitro.*

L'invention concernent par ailleurs un anticorps monoclonal, ou un fragment de liaison de l'antigène de celui-ci, qui fixe spécifiquement l'hétérocarpine.

L'hétérocarpine a pour propriété de fixer le GHRH humain. *In vitro*, l'hétérocarpine fixe le GHRH humain et inhibe ainsi la synthèse d'AMP cyclique induite lors de la fixation du GHRH humain sur son récepteur. *In vivo,* chez le rat, le complexe hétérocarpine/GHRH humain se forme dans le compartiment sanguin et inhibe de manière dose-dépendante la synthèse de GH induite par 10 µg de GHRH humain dans un rapport mole à mole. L'hétérocarpine a pour propriété de fixer le GHRH humain.

Ces propriétés rendent ces composés aptes à une utilisation pharmaceutique. L'invention a donc également pour objet, à titre de médicament, l'hétérocarpine sous une forme glycosylée ou non glycosylée. Elle concerne aussi des compositions pharmaceutiques contenant, à titre de principe actif, l'hétérocarpine sous une forme glycosylée ou non glycosylée, ladite composition comprenant aussi un ou des excipients pharmaceutiquement acceptables. Elle a de plus pour objet l'utilisation de l'hétérocarpine sous une forme glycosylée ou non glycosylée pour préparer des médicaments destinés à antagoniser les effets de GHRH, à traiter les maladies prolifératives (et notamment le cancer), à traiter l'acromégalie ou à traiter les rétinopathies et les néphropathies diabétiques. En ce qui concerne le cancer, l'hétérocarpine sera particulièrement adaptée pour préparer un médicament destiné à traiter les tumeurs carcinoïdes et pancréatiques, les gangliocytomes hypothalamohypophysaires, les carcinomes bronchiques, intestinaux et hépatiques, les tumeurs sympathoadrénergiques, les phéochromocytomes, les adénomes hypophysaires et les carcinomes thyroïdiens. L'hétérocarpine sera particulièrement adaptée pour préparer un médicament destiné à traiter les cancers dont la croissance est dépendante du facteur de croissance GHRH, et notamment pour préparer un médicament destiné à traiter un cancer choisi parmi le cancer du poumon à petites cellules et le cancer du sein (et tout particulièrement le cancer du poumon à petites cellules).

Est également décrit en tant que médicament, un anticorps monoclonal, ou un fragment de liaison de l'antigène de celui-ci, qui fixe spécifiquement l'hétérocarpine. Elle concerne de plus une composition pharmaceutique comprenant, à titre de principe actif, un anticorps monoclonal, ou un fragment de liaison de l'antigène de celui-ci, qui fixe spécifiquement l'hétérocarpine, ladite composition comprenant aussi un ou des excipients pharmaceutiquement acceptables. Elle concerne en outre l'utilisation d'un anticorps monoclonal, ou d'un fragment de liaison de l'antigène de celui-ci, qui fixe spécifiquement l'hétérocarpine, pour préparer des médicaments destinés à antagoniser les effets de GHRH, à traiter les maladies prolifératives (et notamment le cancer), à traiter l'acromégalie ou à traiter les rétinopathies et les néphropathies diabétiques. En ce qui concerne le cancer, ledit anticorps monoclonal ou ledit fragment de liaison de l'antigène de celui-ci sera particulièrement adapté pour préparer un médicament destiné à traiter les tumeurs carcinoïdes et pancréatiques, les gangliocytomes hypothalamohypophysaires, les carcinomes bronchiques, intestinaux et hépatiques, les tumeurs sympathoadrénergiques, les phéochromocytomes, les adénomes hypophysaires et les carcinomes thyroïdiens.

L'utilisation de l'hétérocarpine comme excipient dans une composition pharmaceutique destinée à la libération prolongée de GHRH est également décrite, ainsi qu'une composition pharmaceutique comprenant GHRH, de l'hétérocarpine et un ou des excipients pharmaceutiquement acceptables.

Sont enfin décrits des procédés permettant d'extraire et d'isoler l'hétérocarpine à partir de cellules de la plante *Pilocarpus Heterophyllus,* lesdites cellules provenant de préférence de cultures *in vitro*. Ces procédés comprennent essentiellement une étape d'extraction des cellules de la plante *Pilocarpus Heterophyllus* avec de l'eau à une température de 0 à 50 °C, et de préférence de 4 à 25 °C, ladite étape d'extraction étant suivie d'une étape de filtration afin de séparer le filtrat riche en hétérocarpine des cellules de *Pilocarpus Heterophyllus* et d'une ou plusieurs étapes de séparation de l'hétérocarpine des autres composants extraits de la plante *Pilocarpus Heterophyllus.*

Selon une première variante, ces procédés d'extraction et d'isolement comprennent essentiellement les étapes successives suivantes :
a) une étape d'extraction des cellules de la plante *Pilocarpus Heterophyllus* avec de l'eau à une température de 0 à 50 °C, et de préférence de 4 à 25 °C, ladite étape d'extraction étant suivie d'une étape de filtration afin de séparer le filtrat riche en hétérocarpine des cellules de *Pilocarpus Heterophyllus* ;
b) une étape de précipitation des protéines extraites, par exemple par addition de sulfate d'ammonium, suivie d'une étape de séparation du précipité (par filtration ou, de préférence, par centrifugation) ;
c) la mise en solution du précipité récupéré à l'étape b) dans de l'eau ; et
d) une étape de chromatographie par gel-filtration afin de séparer l'hétérocarpine des autres composants de la solution.

Selon une autre variante, ces procédés d'extraction et d'isolement comprennent essentiellement les étapes successives suivantes :
a) une étape d'extraction des cellules de la plante *Pilocarpus Heterophyllus* avec de l'eau à une température de 0 à 50 °C, et de préférence de 4 à 25 °C, ladite étape d'extraction étant suivie d'une étape de filtration afin de séparer le filtrat riche en hétérocarpine des cellules de *Pilocarpus Heterophyllus* ;
b) une étape de dégraissage de la solution obtenue en a), acidifiée par ajout d'un acide non oxydant (par exemple de l'acide chlorhydrique, de l'acide sulfurique ou de l'acide phosphorique) à un pH de préférence compris entre 2 et 4, à l'aide d'une extraction liquide-liquide (de préférence en utilisant un solvant organique comme le dichlorométhane, l'heptane, l'hexane ou le cyclohexane) ;
c) une étape d'élimination des tannins par mise en contact de la solution dégraissée obtenue en c) avec de la polyvinylpyrrolidone (ou encore du nylon 66) suivie d'une filtration sur résine à pores larges (de préférence une telle résine à base de polystyrènes comme la résine Diaion^{®} HP-20) ;
d) le passage à pH alcalin (de préférence entre pH 9 et 11) du filtrat obtenu après l'étape c) par ajout d'une base comme l'hydroxyde d'ammonium, l'hydroxyde de sodium ou l'hydroxyde de potassium ;
e) une ou des étapes de filtration sur résine échangeuse d'anions, l'éluant pour cette ou ces étapes de filtration étant de préférence une solution tampon ayant un pH entre 9 et 11 et contenant éventuellement des gradients de concentration en un sel (comme par exemple le chlorure de sodium ou le sulfate d'ammonium), afin de séparer l'hétérocarpine des autres composants de la solution ; et
f) une étape de dessalage consistant en le passage de la solution obtenue à l'étape e) sur une résine séparant les constituants d'un mélange en fonction de leur masse moléculaire (comme la résine Sephadex^{®} G25 ou Superdex^{®} 200 HR) et l'élution de ce mélange sur ladite résine avec de l'eau.

Les compositions pharmaceutiques utilisées dans l'invention peuvent être sous forme solide comme, par exemple, les poudres, pilules, granules, comprimés, liposomes, gélules ou suppositoires. Les pilules, les comprimés ou les gélules peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré localement, par exemple à l'emplacement même d'une tumeur. Le composé peut aussi être administré selon un processus de libération prolongée (par exemple en utilisant une composition à libération prolongée ou une pompe de perfusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques utilisées dans l'invention peuvent également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'administration d'un médicament selon l'invention pourra se faire par voie topique, orale, parentérale, par injection intramusculaire, etc.

La dose d'un composé, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

Conformément à l'invention, on peut préparer l'hétérocarpine par le procédé décrit ci-après.

### Préparation de l'hétérocarpine

Selon une variante préférée de l'invention, des cultures *in vitro* de cals ou de suspensions cellulaires issus de différents organes de la plante ont été effectuées. Ces tissus cultivés sur milieu semi-solide ou liquide sont capables de bio-synthétiser des composés ayant des propriétés biologiques.

Par « cal », il faut entendre dans la présente demande un amas macroscopique de cellules indifférenciées de plantes en culture sur un milieu nutritif semi-solide. Par « cellules indifférenciées » sont désignées dans la présente demande des cellules qui ont une aptitude sous certaines conditions à se multiplier sous forme d'un cal ou d'une suspension cellulaire sans phénomène de morphogenèse. Enfin, par « suspension cellulaire », on entend des cellules indifférenciées pouvant former des amas microscopiques en culture dans un milieu de nutrition liquide.

Le choix du milieu nutritif, des hormones, des conditions de culture font partie intégrante de l'invention ainsi que l'extraction et l'analyse de l'extrait à partir de ces cultures *in vitro.*

Les cellules de graines de *Pilocarpus Heterophyllus* peuvent être cultivées en suspension par exemple selon la procédure ci-après.

Les organes sont décontaminés selon les méthodes habituelles avant la mise en culture. Des organes de plantules *in vitro* ont également servi de matériel de départ à la callogénèse sans nécessiter de désinfection préalable. Le milieu nutritif de base préféré est l'un des milieux couramment utilisés pour la culture *in vitro :* il s'agit du milieu de Gamborg (décrit dans Gamborg et coll., Nutrient requirements of suspension cultures of Soybean root cells, Exp. Cell Res. (1968), 50(1), 151-158). La source de carbone est le saccharose mais le glucose peut également être employé à une concentration de 1 à 120 g/l, de préférence de 30 g/l environ. On peut également diminuer la teneur en macro-éléments par un facteur 2. Le milieu est additionné d'auxine ou d'une auxine et d'une cytokinine avec une préférence pour l'association des 2 hormones, en général l'acide 2,4-dichlorophénoxyacétique et la kinétine, mais l'acide α-naphtalèneacétique (ANA), l'acide β-indoleacétique (AIA), l'acide β-indolbutanoïque (AIB) ou le picloram peuvent aussi être combinés à la kinétine ou à la benzylaminopurine (BAP). La concentration peut varier de 0,1 à 10 mg/l pour l'auxine (on pourra choisir par exemple 1 mg/l), et de 0,01 à 2 mg/l pour la cytokinine (on pourra choisir par exemple 0,06 mg/l). Les vitamines sont celles associées aux différents milieux de base. Les cultures sont faites à la lumière ou à l'obscurité. La température peut varier de 10 °C à 33 °C mais sera préférentiellement d'environ 23 °C. Le pH du milieu est compris entre 4 et 6,5 et préférentiellement ajusté à 5,8 avant stérilisation. Le milieu peut par ailleurs être additionné ou non d'agar.

Les cals primaires apparaissent après quelques jours de culture et peuvent être séparés de l'implant d'origine, prélevés et repiqués après environ 1 mois puis cultivés sur milieu semi-solide gélosé (en tube ou en boite de Pétri), avec des passages de 4 à 8 semaines, de préférence 6 semaines, on peut ainsi conserver un cal pendant des années par repiquages successifs sur des milieux neufs. On peut également repiquer le cal dans un milieu de culture liquide agité (fiole erlenmeyer ou bioréacteur) avec des repiquages de 2 à 6 semaines de préférence 3 semaines.

Les souches obtenues se distinguent par l'origine génétique, les conditions de culture, l'aspect et l'absence de morphogénèse.

Les cellules de *Pilocarpus Heterophyllus* lyophilisées sont extraites avec de l'eau à une température de 0 à 50 °C, et de préférence de 4 à 25 °C. L'extrait ainsi obtenu est lyophilisé avant d'être redissous à une concentration adéquate (par exemple environ 30 % de matière sèche). Les protéines précipitées par ajout d'une solution concentrée de sulfate d'ammonium (par exemple à une concentration représentant de 70 à 90 % de la concentration de saturation) sont dissoutes dans un minimum d'eau et les matières insolubles sont récupérées par centrifugation. Les protéines sont ensuite séparées par chromatographie sur colonne (l'éluant étant de préférence de l'eau) et l'hétérocarpine (identifiable par sa masse moléculaire d'environ 90,9 kDa) peut alors être récupérée.

### Préparation d'anticorps fixant spécifiquement l'hétérocarpine

La présente demande décrit des agents de fixation, comme les anticorps qui fixent spécifiquement l'hétérocarpine. Un tel agent est dit comme « fixant spécifiquement » une protéine s'il réagit à un niveau détectable (par exemple par un essai ELISA) avec ladite protéine et ne réagit pas de manière détectable avec d'autres protéines. « La fixation » se réfère à une association non covalente entre 2 molécules séparées de telle sorte qu'un complexe se forme. La capacité à la fixation peut être évaluée, par exemple, par la détermination de la constante de fixation pour la formation du complexe. La constante de fixation est la valeur obtenue lorsque la valeur de la concentration du complexe est divisée par le produit des valeurs des concentration des composants non complexés. 2 produits seront dits « fixés » lorsque la constante de fixation atteint 103 l/mol. La constante de fixation peut être déterminée en utilisant des méthodes bien connues de l'homme du métier.

N'importe quel agent capable de répondre aux critères ci-dessus peut être considéré comme un agent fixant.

Dans la présente demande, un agent de fixation est de préférence un anticorps ou un fragment de celui-ci. Les anticorps peuvent être préparés par n'importe quelle technique disponible à l'homme du métier (cf. Harlow et Lane, Antibodies: A Laboratory Manual, Cold Spring Harbor Laboratory, 1988). En général, les anticorps peuvent être produits par des techniques de culture cellulaire incluant la génération d'anticorps monoclonaux ou *via* des transfections de gènes d'anticorps dans des cellules hôtes de bactéries ou de mammifères afin de produire les anticorps recombinants.

Parmi d'autres techniques, on préférera employer celles décrites ci-après. Un immunogène contenant l'hétérocarpine est injecté chez un groupe de mammifères (par exemple des souris, rats, lapins, moutons ou chèvres). Dans cette étape, l'hétérocarpine peut servir d'immunogène sans modification. Alternativement, une réponse immunitaire supérieure peut être induite si l'hétérocarpine est jointe à une protéine de transport comme l'albumine de sérum bovin ou l'hémocyanine de patelle. L'immunogène est injecté chez l'animal hôte, de préférence selon un schéma prédéterminé, et les animaux sont saignés périodiquement. Des anticorps poly-clonaux spécifiques de l'hétérocarpine peuvent ainsi être purifiés à partir de tels antisérum, par exemple, par chromatographie d'affinité en utilisant de l'hétérocarpine couplée à un support solide adéquat.

### Compositions pharmaceutiques destinées à la libération de GHRH :

Ces compositions peuvent notamment être préparées à partir de l'hétérocarpine et de GHRH selon l'une des méthodes décrites dans la revue de De Wolf et Brett, Pharmacological Reviews (2000), 52, 207-236 et les références qui y sont citées.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### OBTENTION DE L'HETEROCARPINE

### Exemple 1 :

### Culture de cellules in vitro :

Une graine de *Pilocarpus Heterophyllus* est mise à germer et la tige issue de cette germination est prélevée. Ladite tige est mise en culture dans un milieu de Gamborg (Gamborg et coll., Nutrient requirements of suspension cultures of Soybean root cells, Exp. Cell Res. (1968), 50(1), 151-158) additionné de 30 g/l de saccharose, de 1 mg/l d'acide 2,4-dichlorophénoxyacétique et de 0,06 mg/l de kinétine. La culture est effectuée dans des tubes à une température de 23° C et dans l'obscurité. Des repiquages sont effectués toutes les 6 semaines dans des conditions habituelles. Les souches, d'aspect granuleux, possèdent une pigmentation beige.

Une cinétique de croissance des souches, basée sur l'augmentation de masse de matière fraîche et sèche de la biomasse, a été réalisée sur 8 semaines. Les cals de 2 tubes sont réunis et constituent une récolte bihebdomadaire, la première récolte ayant lieu au temps 0. Cals et gélose sont ensuite récoltés et lyophilisés. On constate que la croissance est exponentielle jusqu'à 6 semaines de culture avant l'apparition d'une phase stationnaire de croissance.

### Extraction des cultures cellulaires :

25 g de cellules de *Pilocarpus Heterophyllus* lyophilisées sont extraites 2 fois par immersion dans 375 ml d'eau à 4° C, et laissées la nuit à 4° C, puis dans 250 ml d'eau à 4° C pendant 4 heures et finalement lavées avec 125 ml d'eau à 4° C. Chaque solution aqueuse ainsi obtenue est filtrée sous vide au travers d'un filtre de verre surmonté de célite pour séparer les débris cellulaires de la solution aqueuse. Les solutions aqueuses ainsi combinées sont ensuite lyophilisées pour obtenir 9,4 g de matière sèche. L'extrait sec lyophilisé est ensuite dissous dans 31 ml d'eau à 20° C pour obtenir une solution contenant 30 % d'extrait sec. 17,4 g de sulfate d'ammonium sont ajoutés par petites portions avec une agitation magnétique constante pour précipiter la fraction protéique. Le précipité protéique est ensuite séparé de la solution de sulfate d'ammonium par centrifugation à 3000 rpm pendant 20 minutes. La solution de sulfate d'ammonium est décantée et les protéines précipitées sont dissoutes dans 22 ml d'eau, re-centrifugées et filtrées pour éliminer les particules insolubles.

Le filtrat obtenu est ensuite soumis à une chromatographie par gel-filtration. Il est injecté dans une colonne (Buchi N° 19678, L = 230 mm ; diamètre interne = 26 mm) remplie de Superdex ^{™} 200 (Amersham Pharmacia Biotech, référence n° 17-1043-01 ; particules de diamètre moyen de 13 µm) préparée selon les recommandations du fabricant en utilisant de l'eau ultra-pure (Water's Milli-Q) comme éluant à un débit de 5 ml par minute. Des fractions de 40 ml sont ainsi collectées et la protéine active est trouvée dans la troisième et la quatrième fraction. Ces fractions sont lyophilisées pour obtenir environ 14,2 mg de produit actif.

La pureté du produit obtenu est démontrée par l'apparition d'une seule bande sur gel d'électrophorèse contenant du dodécylsulfate de sodium (SDS PAGE). Le produit correspondant à cette bande est désigné dans ce qui suit comme l'hétérocarpine.

### Exemple 2 :

Les cellules cultivées *in vitro* selon la même procédure que celle décrite dans l'exemple 1 ci-dessus sont extraites selon la méthode décrite ci-après.

100 g de cellules de *Pilocarpus Heterophyllus* lyophilisées sont extraites à l'aide de 2 litres d'eau déminéralisée à 20 °C, le mélange étant maintenu agité pendant une nuit. Les cellules et l'extrait sont filtrés par succion sur fritté (porosité 3, diamètre de 20 cm) recouvert d'un lit de célite (préalablement lavée avec de l'acide ; 1 à 2 cm d'épaisseur). Les cellules récupérées sont lavées avec 400 ml d'eau déminéralisée avant d'être éliminées. Le filtrat aqueux est ensuite acidifié à pH 3,0 par addition d'environ 10 ml d'acide chlorhydrique à 18%. La solution acidifiée est ensuite dégraissée par extraction liquide-liquide à l'aide de 400 ml de dichlorométhane. La phase dichlorométhane est décantée puis éliminée. La solution dégraissée est soumise à une évaporation rotative pour éliminer le dichlorométhane résiduel. Environ 30 g de polyvinylpyrrolidone sont ensuite ajoutés à la solution dégraissée (pH environ 3,0) et le mélange est agité pendant environ 30 minutes pour éliminer les tannins. Le mélange est filtré un lit par succion sur fritté (porosité 3, diamètre de 10 cm) recouvert d'un lit mixte composé de 25 g de célite (préalablement lavée avec de l'acide) et 25 g de polyvinylpyrrolidone. Le filtrat est ensuite passé à travers un lit de 400 ml de Diaion^{®} HP-20 (Mitsubishi Chemical Company) pré-activé selon les instructions du fabricant. Le filtrat résultant est ensuite rendu alcalin (pH 10) par addition d'environ 60 ml d'une solution d'hydroxyde d'ammonium à 20%. Une légère précipitation apparaît après 30 minutes de repos. 1 g de célite (préalablement lavée avec de l'acide) est ajouté à la solution alcaline qui est ensuite filtrée par succion à travers un filtre à membrane (0,22 µm). Environ 2 litres de filtrat sont ensuite passés à travers une colonne HiPrep^{®} Q XL 16/10, montée sur un purificateur Akta^{®} et pré-équilibrée à pH 10,2 avec un tampon pipérazine/HCl 0,1*M*, avec un débit de 0,5 ml par minute (la colonne HiPrep^{®} et le purificateur Akta^{®} sont tous deux des produits de la société Amersham Biosciences). La colonne est ensuite successivement lavée avec 6 volumes de colonne du tampon de départ à pH 10,2, 5 volumes de colonne du même tampon contenant une concentration 0,2*M* de NaCl; et 10 volumes de colonne du même tampon contenant une concentration 1*M* de NaCl. La majorité de l'hétérocarpine est récupérée dans les trois premiers 3 volumes de colonne de tampon contenant la concentration 1*M* de NaCl. Les fractions actives sont dessalées par passage à travers une colonne Sephadex^{®} G25 (volume du lit : 260 ml) en utilisant de l'eau déminéralisée comme éluant. Les fractions actives, trouvées dans le premier volume de colonne correspondant au volume mort, sont ensuite lyophilisées pour obtenir 170 mg d'hétérocarpine. L'hétérocarpine ainsi obtenue est pratiquement mono-bande sur gel SDS PAGE.

### CARACTERISATION DE L'HETEROCARPINE

### Analyse et micro-séquençage :

Les échantillons sont chargés sur un gel de polyacrylamide à 10 %. Après migration, les gels sont fixés et colorés au bleu de Coomassie.

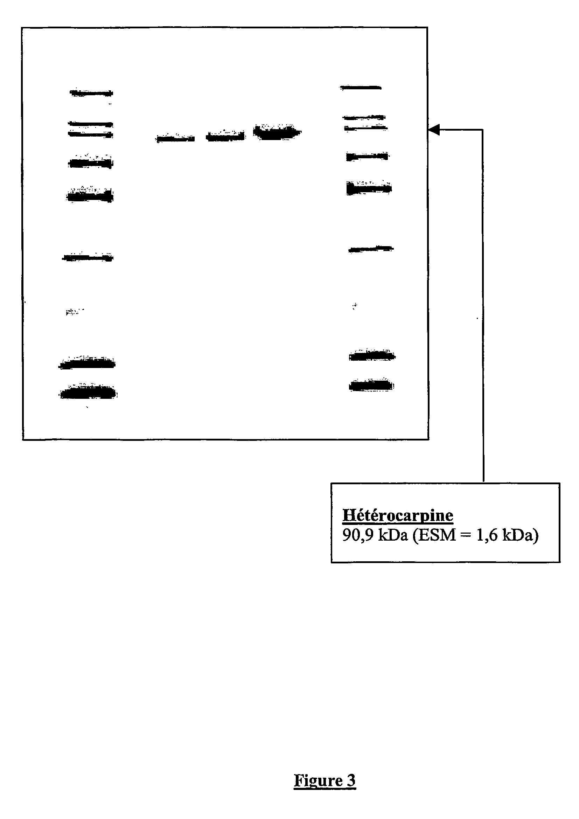

Les pistes du gel représenté dans la **Figure 3** correspondant aux pistes 1, 2, 3, 4 et 5 sont respectivement le marqueur de poids moléculaire (Amersham), 0,5, 1 et 2 µg du contenu de la fraction finale d'hétérocarpine telle qu'obtenue à l'exemple 1 et le marqueur de masse moléculaire (Amersham). La détermination de la masse moléculaire grâce à une courbe standard du marqueur de masse moléculaire en utilisant des outils informatiques classiques et bien connus de l'homme de l'art (par exemple, le logiciel Bio-Profil BiolD de Viber Lourmat) permet de montrer que l'hétérocarpine possède une masse moléculaire de 90,9 kiloDaltons (± 1,6 kiloDaltons).

Pour l'analyse par micro-séquençage de protéine, la bande de polyacrylamide contenant la protéine est découpée et digérée dans 300 µl de tampon de digestion contenant 50 mM Tris (pH 8,6), 0,03 % de dodécylsulfate de sodium à 35° C pendant 18 heures en présence de 0,4 µg d'endolysine-C (Sigma). Les peptides obtenus sont séparés, par HPLC, sur colonne en ligne de DEAE-C18 de 1 mm de diamètre. Le gradient de séparation est basé sur un mélange d'acétonitrile (de 2 à 70 %) et d'acide trifluoroacétique à 0,1 % (TFA). Le séquençage est ensuite réalisé sur un séquenceur Procise (Applied Biosystem). Trois pics ont ainsi été séquencés, permettant de caractériser de manière unique l'hétérocarpine. Les séquences correspondantes sont identifiées dans la présente demande par SEQ. ID. NO. 1, SEQ. ID. NO. 2 et SEQ. ID. NO. 3.

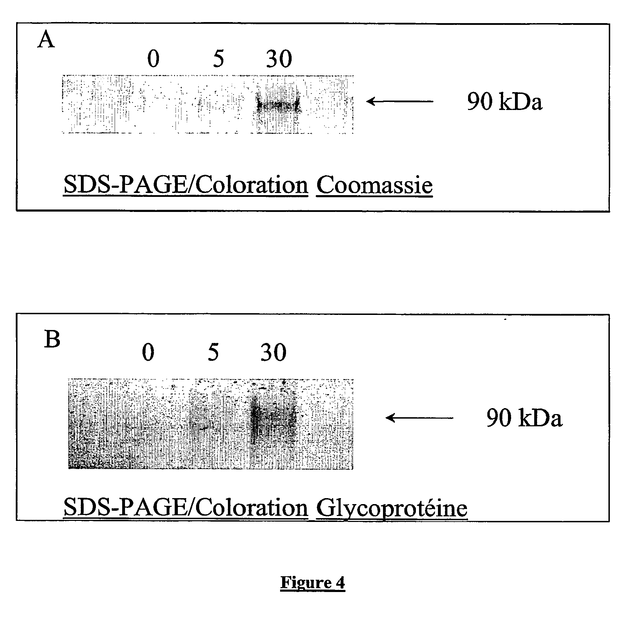

L'analyse des glycoprotéines est réalisée par la détection de structures sucrées des glycoprotéines séparées par gel SDS-PAGE. Ce système de détection est une modification des méthodes « Periodic acid-Schiff » et conduit à l'apparition de bandes magenta mettant en évidence les glycoprotéines (Sigma). On obtient, pour l'hétérocarpine telle qu'obtenue à l'exemple 1, le résultat reproduit en Figure 4.

### PROPRIETES PHARMACOLOGIQUES DE L'HETEROCARPINE

### Transfections stables du récepteur humain à GHRH (hGHRH-R) :

Les cellules humaines embryonnaires de reins, HEK-293, (une lignée cellulaire développée par le Dr. Stuart Sealfon, Mount Sinai Medical School, New York, New York) exprimant de manière stable le récepteur humain à GHRH ont été obtenues du Dr. Kelly Mayo (Northwestern University, Chicago, IL).

### Culture cellulaire et préparation membranaire :

Les cellules HEK-293 transfectées de manière stable avec le récepteur humain à GHRH décrites ci-dessus sont cultivées en DMEM (milieu de Eagle modifié par Dulbecco, forte teneur en glucose ; fourni par Life technologies) supplémenté avec 0,4 mg/ml de G418 (Life technologies) en présence de 10 % de sérum de veau foetal et de 4 mM de L-glutamine (Life technologies). Les cellules sont homogénéisées dans le tampon A contenant 50 mM HEPES (pH 7,4), 5 mM de chlorure de magnésium (MgCl₂), 2 mM d'acide éthylèneglycol-bis(2-amino-éthyl)-N,N,N',N'-tétraacétique (EGTA) et 50 µg/ml de bacitracine puis sont soumises à sonication dans le même tampon A. Les cellules ainsi homogénéisées sont centrifugées à 4° C à 39 000 g pendant 10 minutes, suspendues dans le tampon A et re-centrifugées à 4° C à 40 000 g pendant 10 minutes.

Les protéines totales membranaires sont quantifiées par la technique de Bradford. Les membranes culottées sont ainsi stockées à -80° C pour une utilisation ultérieure.

### Test de liaison compétitive sur hGHRH-R :

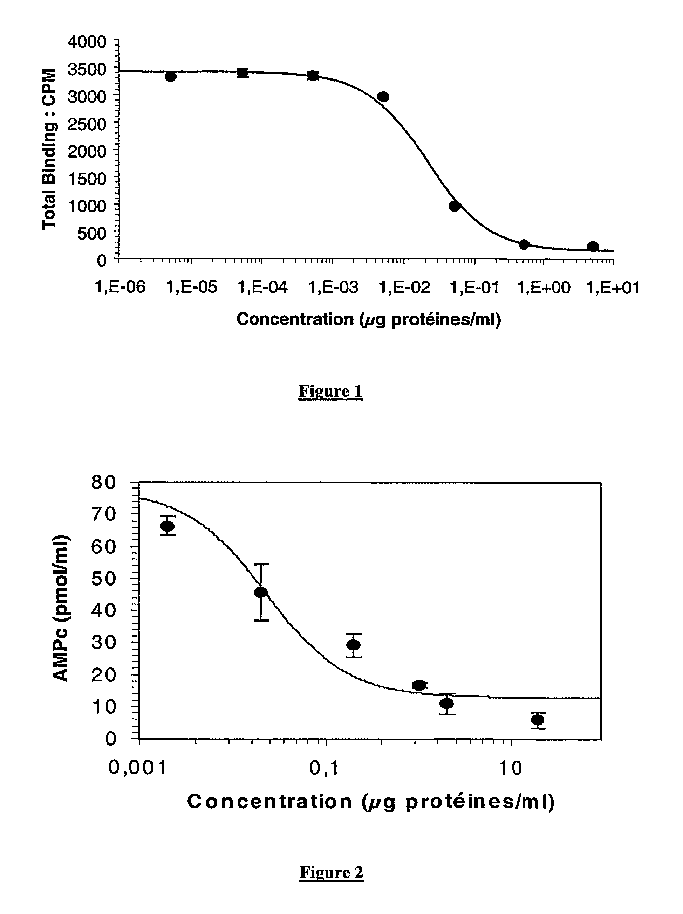

Les membranes des cellules HEK-293 transfectées de manière stable avec le récepteur humain à GHRH sont diluées à la concentration de 100 µg/ml dans le tampon réactionnel contenant 50 mM HEPES (pH 7,4), 5 mM de MgCl₂, 2 mM d'EGTA, 50 µg/ml de bacitracine et 0,5 % d'albumine de sérum bovin (BSA). Les membranes sont incubées avec 0,05 nM de [¹²⁵I]GHRH(1-44 amide) (Amersham) dans un volume final de 200 µl en présence de concentrations croissantes d'hétérocarpine pendant 2 heures à 23° C. La réaction est arrêtée par une filtration rapide sur des filtres 96 puits GF/C pré-chargés à 0,1 % en polyéthylènimine. Les filtres sont ensuite lavés trois fois à 4° C avec du tampon de lavage contenant 50 mM Tris (pH 7,4) en utilisant une station de filtration Packard 96 puits. Les filtres ainsi séchés sont submergés de 20 µl de cocktail scintillant (Microscint O, Packard) et sont soumis à un comptage sur le Topcount (Packard). L'activité non-spécifique est déterminée en présence de 100 nM de hGHRH. Une courbe dose-réponse est générée pour hGHRH (0,001 nM-100 nM) et les résultats obtenus sont repris en **Figure 1****.**

### Formation compétitive d'AMP cyclique :

Les cellules HEK-293 transfectées de manière stable avec le récepteur humain à GHRH sont distribuées dans des plaques de culture 48 puits et cultivées pendant 3 jours. Le milieu de culture est ensuite retiré et remplacé par le milieu B contenant 250 µl de DMEM (milieu de Eagle modifié par Dulbecco, forte teneur en glucose ; fourni par Life technologies) en présence de 0,5 % de BSA, 0,5 mM de 3-isobutyl-1-méthylxanthine (IBMX) et pré-incubées 5 minutes à 37° C. A la fin de la période de pré-incubation, l'hétérocarpine est testée pendant 20 minutes additionnelles. Les concentrations observées sont reportées en **Figure 2****.** L'incubation est stoppée par l'ajout de 100 µl de HCl 0,1M et les aliquots sont analysés pour leur contenu en AMP cyclique en utilisant le kit FlashPlate (New England Nuclear).

### Dosage de GH chez les rats :

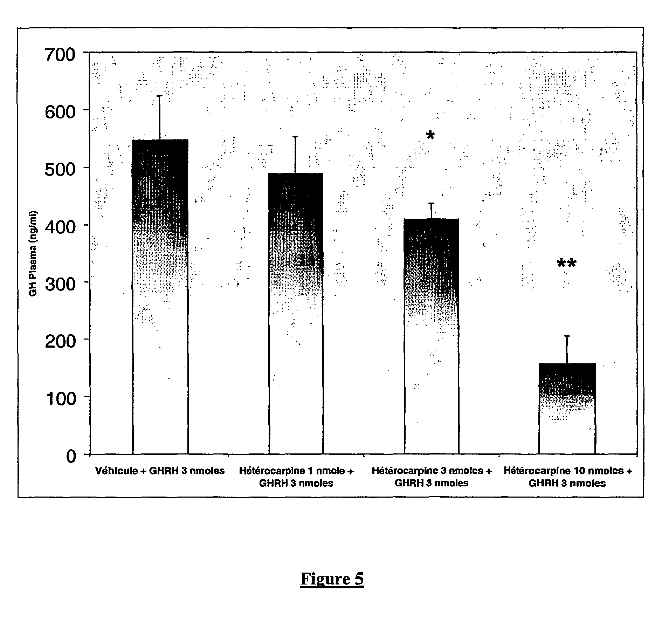

Les niveaux de GH chez les rats (mâles, Sprague Dawley) sont mesurés dans des prélèvements sanguins par un test enzymo-immunologique développé par Spi-Bio (Spi-Bio, France). Les rats sont traités par injection intra-veineuse d'hétérocarpine à des doses croissantes (véhicule seul, 1, 3 et 10 nmol), puis, 10 minutes après, par injection intra-veineuse de 10 µg (3 nmol) de hGHRH. Dix minutes après l'injection du hGHRH, les niveaux de l'hormone de croissance sont mesurés dans les prélèvements sanguins comme décrit ci-dessus. Les résultats obtenus sont représentés en Figure 5.

### Mesure de l'activité anti-tumorale :

Les cellules tumorales humaines et en particulier les cellules de cancer du poumon à petites cellules H-69 sont injectées sous la peau de souris athymiques afin de produire une xénogreffe de tumeur humaine d'environ 80 mm³ une dizaine de jours après la première greffe. Les souris sont traitées tous les deux jours par injection intra-veineuse d'hétérocarpine à des doses croissantes (véhicule seul, 2,5 mg/kg, 5 mg/kg et 10 mg/kg). Le volume des tumeurs est ensuite mesuré tous les 4 jours pendant toute la durée du traitement.

### Brève description des figures :

La **Figure 1** est une courbe représentant l'inhibition de fixation du GHRH humain sur le récepteur à GHRH humain en fonction de concentrations croissantes d'hétérocarpine.
La **Figure 2** est une courbe représentant l'inhibition de production d'AMP cyclique dans des cellules transfectées de manière stable avec le récepteur à GHRH humain en présence de 10 nM de GHRH humain en fonction de concentrations croissantes d'hétérocarpine.
La **Figure 3** est la reproduction d'une plaque de gel de protéine SDS-PAGE montrant la présence de l'hétérocarpine ayant un poids moléculaire de 90,9 kDa.
La **Figure 4** est la reproduction d'une plaque de gel de protéine SDS-PAGE montrant que l'hétérocarpine est une glycoprotéine (Panneau B).
La **Figure 5** est une représentation sous forme d'histogrammes représentant l'inhibition de synthèse de GH chez le rat en présence de 10 µg de GHRH humain en fonction de concentrations croissantes d'hétérocarpine.

## Revendications

1. Utilisation d'une protéine isolée susceptible d'être obtenue par extraction de la plante *Pilocarpus heterophyllus*, ladite protéine étant **caractérisée en ce qu'**elle possède une masse moléculaire d'environ 90,9 kDa et comporte les fragments de séquences peptidiques SEQ. ID. NO. 1, SEQ. ID. NO. 2 et SEQ. ID. NO. 3, cette protéine étant en outre susceptible de se présenter sous une forme glycosylée ou non glycosylée, pour préparer un médicament destiné à traiter les cancers dont la croissance est dépendante du facteur de croissance GHRH et choisis parmi le cancer du poumon à petites cellules et le cancer du sein.

2. Utilisation selon la revendication 1, **caractérisée en ce que** la protéine a été obtenue à partir d'un extrait des cellules de la plante *Pilocarpus Heterophyllus* cultivées *in vitro.*

3. Utilisation selon la revendication 1 ou 2, **caractérisée en ce que** le cancer dont la croissance est dépendante du facteur de croissance GHRH est le cancer du poumon à petites cellules.

4. Utilisation selon la revendication 1 ou 2, **caractérisée en ce que** le cancer dont la croissance est dépendante du facteur de croissance GHRH est le cancer du sein.

## Claims

1. Use of an isolated protein which can be obtained by extraction from the plant *Pilocarpus heterophyllus*, said protein being **characterized in that** it has a molecular mass of approximately 90.9 kDa and comprises fragments of peptide sequences SEQ.ID.NO.1, SEQ.ID.NO.2 and SEQ.ID.NO.3; said protein moreover being able to be presented in a glycosylated or non-glycosylated form, for preparing a medicament intended to treat cancers the growth of which is dependent on the growth factor GHRH.

2. Use according to claim 1, **characterized in that** the protein has been obtained from an extract from cells of the plant *Pilocarpus Heterophyllus* cultured *in vitro.*

3. Use according to claim 1 or 2, **characterized in that** the cancer the growth of which is dependent on the growth factor GHRH is small-cell lung cancer.

4. Use according to claim 1 or 2, **characterized in that** the cancer the growth of which is dependent on the growth factor GHRH is breast cancer.

## Patentansprüche

1. Verwendung eines isolierten Proteins, das durch Extraktion der Pflanze *Pilocarpus heterophyllus* erhalten werden kann und das **dadurch gekennzeichnet ist, dass** es eine Molmasse von etwa 90,9 kDa besitzt und die Peptidsequenzfragmente SEQ.ID.NO.1, SEQ.ID.NO.2 und SEQ.ID.NO.3 umfasst, wobei dieses Protein außerdem in einer glycosylierten oder nicht glycosylierten Form vorliegen kann, für die Herstellung eines Medikaments, das zur Behandlung von Krebsarten bestimmt ist, deren Wachstum von dem Wachstumsfaktor GHRH abhängig ist und die aus kleinzelligem Lungenkrebs und Brustkrebs ausgewählt sind.

2. Verwendung nach Anspruch 1, **dadurch gekennzeichnet, dass** das Protein aus einem Extrakt der *in vitro* kultivierten Zellen der Pflanze *Pilocarpus heterophyllus* erhalten wurde.

3. Verwendung nach Anspruch 1 oder 2, **dadurch gekennzeichnet, dass** der Krebs, dessen Wachstum von dem Wachstumsfaktor GHRH abhängig ist, kleinzelliger Lungenkrebs ist.

4. Verwendung nach Anspruch 1 oder 2, **dadurch gekennzeichnet, dass** der Krebs, dessen Wachstum von dem Wachstumsfaktor GHRH abhängig ist, Brustkrebs ist.


### LISTE DE SEQUENCES

<110> Société de Conseils de Recherches et d'Application
<120> L'hétérocarpine, une protéine d'origine végétale aux propriétés anticancéreuses
<130> RS 329 FR - Liste séquences
<140> FR 02/15560
   <141> 2002-08-26
<160> 3
<170> PatentIn Ver. 2.1
<210> 1
   <211> 10
   <212> PRT
   <213> Pilocarpus Heterophyllus
<400> 1
   Lys Leu Ile Gly Ala Arg Tyr Phe Asp Lys
    1               5                  10
<210> 2
   <211> 14
   <212> PRT
   <213> Pilocarpus Heterophyllus
<400> 2
   Tyr Gly Glu Asp Ile Ile Val Gly Val Ile Asp Ser Gly Val
    1               5                  10
<210> 3
   <211> 6
   <212> PRT
   <213> Pilocarpus Heterophyllus
<400> 3
   Pro Glu Ser Glu Ser Tyr
    1               5